# EUROPEAN PATENT SPECIFICATION

(11) **EP 0 964 845 B1**
(45) Date of publication and mention of the grant of the patent: **28.07.2004**
(21) Application number: 98905114.9
(22) Date of filing: 18.02.1998
(51) Int. Cl.: C07C 17/00, C07C 21/00

(54) **PROCESSES FOR THE MANUFACTURE OF 1,1,1,3,3-PENTAFLUOROPROPENE, 2-CHLORO-PENTAFLUOROPROPENE**
VERFAHREN FÜR DIE HERSTELLUNG VON 1,1,1,3,3-PENTAFLUORPROPEN UND 2-CHLOR-PENTAFLUORPROPEN
PROCEDES DE PRODUCTION DE 1,1,1,3,3-PENTAFLUOROPROPENE, 2-CHLORO-PENTAFLUOROPROPENE

(30) Priority: 19.02.1997 US 38430 P; 16.06.1997 US 49723 P; 25.08.1997 US 56795 P
(43) Date of publication of application: 22.12.1999
(60) Divisional application: 03078647.9
(73) Proprietor: E. I. du Pont de Nemours and Company, Wilmington, Delaware 18898 (US)
(72) Inventor: MANOGUE, William, H., Newark, DE 19711 (US); RAO, V., N., Mallikarjuna, Wilmington, DE 19809 (US); SIEVERT, Allen, Capron, Elkton, MD 21921 (US); SWEARINGEN, Steven, H., Wilmington, DE 19801 (US)
(74) Representative: Carpmaels & Ransford
(86) International application number: PCT/US1998/003132
(87) International publication number: WO 1998/037043

(56) References cited:
- EP-A- 0 442 075
- EP-A- 0 634 384
- EP-A- 0 676 386
- WO-A-90/08748
- WO-A-95/04022
- WO-A-97/19751
- US-A- 3 505 417
- US-A- 3 636 173

## Description

### FIELD OF THE INVENTION

This invention relates to fluorine-substituted hydrocarbons, and more particularly to processes for producing CF₃CH=CF₂, CF₃CCl=CF₂ and saturated derivatives thereof such as CF₃CH₂CF₃, CF₃CH₂CHF₂ and CF₃CHFCF₃.

### BACKGROUND

A number of chlorine-containing halocarbons are considered to be detrimental toward the Earth's ozone layer. There is a world-wide effort to develop materials having lower ozone depletion potential that can serve as effective replacements. For example, the hydrofluorocarbon, 1,1,1,2-tetrafluoroethane (HFC-134a) is being used as a replacement for dichlorodifluoromethane (CFC-12) in refrigeration systems. The production of hydrofluorocarbons (i.e., compounds containing only carbon, hydrogen and fluorine), has been the subject of considerable interest to provide environmentally desirable products for use as solvents, blowing agents, refrigerants, cleaning agents, aerosol propellants, heat transfer media, dielectrics, fire extinguishants and power cycle working fluids (see e.g., PCT International Publication No. WO 93/02150).

WO 90/08748 discloses the hydrogenolysis/dehydrohalogenation of certain fluorohalocarbons or fluorohalohydrocarbons with hydrogen in the presence of a rhenium-containing catalyst, which may, optionally contain at least one metal from group VIII of the periodic table (e.g., palladium, platinum, ruthenium or rhodium). The reaction of CF₃CCl₂CF₃ to produce CF₃CHClCF₃ as the major product is illustrated.

U.S. Patent No. 3,505,417 and related U.S. Patent No. 3,636,173 disclose the hydrodehalogenation of certain fluorohalocarbons with hydrogen in the presence of a catalytic composition containing aluminum fluoride and at least one metal (or compounds or mixtures thereof), selected from group I to group VIII of the periodic table. The advantageous use of fluorided alumina in combination with metal phosphate is disclosed in U.S. Patent No. 3,636,173.

### SUMMARY OF THE INVENTION

A process is provided in accordance with this invention for producing pentafluoropropenes of the formula CF₃CX=CF₂, where X is H or Cl. The process comprises hydrodehalogenating CF₃CCl₂CF₃ with hydrogen at an elevated temperature in the vapor phase over a catalyst comprising at least one component selected from the group consisting of elemental metals, metal oxides, metal halides and metal oxyhalides; wherein the metal of said hydrodehalogenation catalyst component is selected from copper, nickel, chromium and mixtures thereof and the halogen of said halides and said oxyhalides is selected from fluorine, chlorine and mixtures thereof.

This invention further provides a process for producing the hydroflurocarbon CF₃CHFCF₃. This process comprises (a) hydrodehalogenating CF₃CCl₂CF₃ with hydrogen as indicated above to produce a product comprising CF₃CCl=CF₂, CF₃CH=CF₂, HCl and HF; and (b) reacting the CF₃CCl=CF₂ produced in (a) with HF to produce CF₃CHFCF₃.

This invention further provides a process for producing the hydrofluorocarbon CF₃CH₂CHF₂. This process comprises (a) hydrodehalogenating CF₃CCl₂CF₃ with hydrogen as indicated above to produce a product comprising CF₃CCl=CF₂, CF₃CH=CF₂, HCl and HF; and (b) reacting at least one of said CF₃CCl=CF₂ and CF₃CH=CF₂ produced in (a) in the vapor phase with hydrogen to produce CF₃CH₂CHF₂.

This invention further provides a process for producing CF₃CH₂CF₃. This process comprises (a) hydrodehalogenating CF₃CCl₂CF₃ with hydrogen as indicated above to produce a product comprising CF₃CCl=CF₂, CF₃CH=CF₂, HCl and HF; and (b) reacting the CF₃CH=CF₂ produced in (a) with HF to produce CF₃CH₂CF₃.

### DETAILED DESCRIPTION

This invention provides a process for producing 1,1,1,3,3-pentafluoropropane (i.e.. CF₃CH₂CHF₂ or HFC-245fa) using 2,2-dichloro-1,1,1,3,3,3-hexafluoropropane (i.e., CF₃CCl₂CF₃ or CFC-216aa).

The present invention includes the hydrodehalogenation of CFC-216aa in a manner which removes a single fluorine from an end carbon while removing at least one chlorine from the internal carbon to produce CF₃CCl=CF₂ (CFC-1215xc) and CF₃CH=CF₂ (HCFC-1225zc). This hydrodehalogenation generally produces a product comprising CF₃CCl=CF₂, CF₃CH=CF₂, HF and HCl, and involves the use of advantageously catalytic components employing copper, nickel and/or chromium. Suitable components include halides such as CuF, CuCl, CuCl₂, CuClF, NiF₂, NiCl₂, NiClF, CrF₃, CrCl₃, CrCl₂F and CrClF₂; oxides such as CuO, NiO, and Cr₂O₃; and oxyhalides such as copper oxyfluoride and chromium oxyfluoride. Oxyhalides may be produced by conventional procedures such as, for example, halogenation of metal oxides.

The catalysts of this invention may contain other components, some of which are considered to improve the activity and/or longevity of the catalyst composition. Preferred catalysts include catalysts which are promoted with compounds of molybdenum, vanadium, tungsten, silver, iron, potassium, cesium, rubidium, barium or combinations thereof. Also of note are chromium-containing catalysts which further contain zinc and/or aluminum or which comprise copper chromite.

The catalyst may be supported or unsupported. Supports such as metal fluorides, alumina and titania may be advantageously used. Particularly preferred are supports of fluorides of metals of Group IIB, especially calcium. A preferred catalyst consists essentially of copper, nickel and chromium oxides (each of said oxides being preferably present in equimolar quantities) preferably promoted with potassium salt, on calcium fluoride.

An especially preferred catalyst contains proportionally 1.0 mole CuO, 0.2 to 1.0 mole NiO, 1 to 1.2 moles Cr₂O₃ on 1.3 to 2.7 moles CaF₂, promoted with 1 to 20 weight%, based on the total catalyst weight, of an alkali metal selected from K, Cs, and Rb (preferably K). When K is the promoter, the preferred amount is from 2 to 15 weight% of the total catalyst.

This catalyst can be prepared by coprecipitating, from an aqueous medium, salts of copper, nickel and chromium (and optionally aluminum and zinc), with and preferably on calcium fluoride; washing, heating and drying the precipitate. An alkali metal compound (e.g., KOH, KF or K₂CO₃) is then deposited on the dried precipitate, followed by calcination to convert the copper, nickel and chromium to the respective oxides. Any soluble copper, nickel and chromium compound may be used, but the chlorides and nitrates are preferred, with the nitrates being especially preferred. Alternatively, promoters such as KOH, KF and K₂CO₃ may be added prior to co-precipitation.

Another group of catalysts which may be used for the conversion of CF₃CCl₂CF₃ contains proportionally 1.0 mole CuO, 0.2 to 1.0 mole NiO, 1 to 1.2 moles Cr₂O₃, 0.4 to 1.0 mole MoO₃, and 0.8 to 4.0 mole CaF₂, optionally promoted with at least one compound from the group consisting of MgF₂, MnF₂, and BaF₂. Palladium or WO₃ may also be present.

The catalyst may be granulated, pressed into pellets, or shaped into other desirable forms. The catalyst may contain additives such as binders and lubricants to help insure the physical integrity of the catalyst during granulating or shaping the catalyst into the desired form. Suitable additives include carbon and graphite. When binders and/or lubricants are added to the catalyst, they normally comprise 0.1 to 5 weight percent of the weight of the catalyst.

The catalyst may be activated prior to use by treatment with hydrogen, air, or oxygen at elevated temperatures. After use for a period of time in the process of this invention, the activity of the catalyst may decrease. When this occurs, the catalyst may be reactivated by treating it with hydrogen, air or oxygen, at. elevated temperature in the absence of organic materials.

The molar ratio of hydrogen to CF₃CCl₂CF₃ fed to the process typically ranges from 1:1 to 30: 1, and is preferably at least 3:1.

The hydrodehalogenation process of CF₃CCl₂CF₃ is suitably conducted at a temperature in the range of from 300°C to 450°C, preferably from 350°C to 400°C. The contact time of reactants with the catalyst bed (i.e., the volume of the catalyst bed divided by the volumetric flow rate at the temperature and pressure of the reaction) is typically from 5 seconds to 4 minutes.

The product from the hydrodehalogenation reaction of CF₃CCl₂CF₃ comprises CF₃CH=CF₂, CF₃CCl=CF₂, HCl and HF and typically other compounds such as unreacted CF₃CCl₂CF₃ and partially reacted compounds such as CF₃CHClCF₃. These products may be separated by conventional means such as distillation and/or decantation and the components may be used individually. For example, CF₃CH=CF₂ (HFC-1225zc) may be used as a co-monomer for producing fluorine-containing polymers. Unreacted starting material (CFC-216aa) can be recycled to the hydrodehalogenation reactor.

Products from the hydrodehalogenation reaction of CF₃CCl₂CF₃ which contain the unreacted CFC-216aa and optionally also contain the partially reacted compound CF₃CHClCF₃, after isolation from the unsaturated hydrodehalogenation products. can also be further reacted with hydrogen (e.g., using a conventional supported palladium hydrogenation catalyst). This hydrogenation can be used to produce CF₃CH₂CF₃ (HFC-236fa), a useful fire extinguishant. If desired, the amount of HFC-236fa produced in this manner can be increased by decreasing the contact time of the CFC-216aa reactant in the hydrodehalogenation reactor.

CF₃CCl=CF₂ (CFC-1215xc) may be reacted with HF to produce CF₃CHFCF₃ (HFC-227ea), which is a useful fire extinguishant. The reaction is typically conducted at an elevated temperature in either the liquid or vapor phase using a fluorination catalyst. For example, CF₃CCl=CF₂ may be reacted with HF in the liquid phase at a temperature of from 100 to 175°C over a pentavalent antimony catalyst (e.g., SbF₅) to produce CF₃CHFCF₃; or CF₃CCl=CF₂ may be reacted with HF in the vapor phase at a temperature of from 300 to 400°C over an unsupported or supported trivalent chromium catalyst (e.g., Cr₂O₃ or Cr₂O₃/AlF₃). Of note are embodiments where the HF produced from the hydrodehalogenation of CF₃CCl₂CF₃ is used for CF₃CHFCF₃ production. Preferably, however, the mole ratio of HF to CF₃CCl=CF₂ used for CF₃CHFCF₃ production is at least 5:1.

The reaction products from the hydrofluorination may be separated by conventional techniques, such as distillation. CF₃CHFCF₃ may form azeotropic combinations with HF and/or HCl; and conventional decantation/distillation may be employed if further purification of CF₃CHFCF₃ is desired.

CF₃CH=CF₂ (CFC-1225zc) may be reacted with HF to produce CF₃CH₂CF₃ (HFC-236fa), which is a useful fire extinguishant. The reaction is typically done in either the liquid or vapor phase with or without using a fluorinating catalyst. For example, CF₃CH=CF₂ may be reacted with HF in the liquid phase at a temperature of from 20°C to 175°C in the absence of a catalyst to produce CF₃CH₂CF₃; or CF₃CH=CF₂ may be reacted with HF in the liquid phase at a temperature of from 0°C to 175°C over a polyvalent metal halide catalyst (e.g., SbF₅, AlF₃, MoF₅, TaF₅, NbF₅, SnCl₄, SbCl₅ or TiCl₄) to produce CF₃CH₂CF₃. Also, CF₃CH=CF₂ may be reacted with HF in the vapor phase at a temperature of from 150°C to 400°C in the absence of a catalyst to produce CF₃CH₂CF₃; or CF₃CH=CF₂ may be reacted with HF in the vapor phase at a temperature of from 50°C to 400°C over an unsupported or supported trivalent chromium catalyst (e.g., Cr₂O₃ or Cr₂O₃/AlF₃) or other vapor phase fluorination catalysts such as AlF₃, carbon, or a transition metal (e.g., Co, Mn, and/or Cr) supported on AlF₃. Of note are embodiments where the HF produced from the hydrodehalogenation of CF₃CCl₂CF₃ is used for CF₃CH₂CF₃ production. Preferably, however, the mole ratio of HF to CF₃CH=CF₂ used for CF₃CH₂CF₃ production is at least 1:1. Reference is made to U.S. Patent No. 5,563,304 for a discussion of vapor phase hydrofluorination.

The reaction products from the hydrofluorination may be separated by conventional techniques, such as distillation. CF₃CH₂CF₃ forms an azeotrope with HF (see U.S. Patent No. 5,563,304) and may also form an azeotrope with HCl; and conventional decantation/distillation may be employed if further purification of CF₃CH₂CF₃ is desired. The azeotropic compositions of CF₃CH₂CF₃ include a composition consisting essentially of from 59 to 37 mole percent HF and from 41 to 63 mole percent CF₃CH₂CF₃ (which forms an azetrope having a boiling point from -25°C at 44 kPa to 100°C at 2900 kPa).

In another embodiment, the HFC-1225zc and/or CFC-1215xc produced by the hydrodehalogenation reaction of this invention may be reacted with hydrogen in the vapor phase to produce CF₃CH₂CHF₂. The reaction of CF₃CCl=CF₂ and/or CF₃CH=CF₂ with hydrogen can employ a hydrogenation catalyst. Suitable hydrogenation catalysts include those which contain a metal (e.g., a Group VIII metal or rhenium). The metal may be supported (e.g., Pd supported on alumina, aluminum fluoride, or carbon) or may be unsupported (e.g., Raney nickel). Carbon-supported metal catalysts are preferred, with Pd/C being particularly preferred. The carbon support is preferably washed with acid prior to depositing the metal on it. Procedures for preparing a catalyst of Group VIII metal or rhenium on an acid-washed carbon support are disclosed in U.S. Patent No. 5,136,113, the entire contents of which are hereby incorporated by reference.

Of note is a process where CF₃CCl=CF₂ and/or CF₃CH=CF₂ is contacted with hydrogen in the presence of a hydrogenation catalyst and in the presence of HCl and HF. The CF₃CH=CF₂ and/or CF₃CCl=CF₂ may be isolated from the hydrodehalogenation reaction effluent by distillation if desired, and then passed to the hydrogenation step, with HCl and HF being separately added in the hydrogenation step. However, it is preferred to pass the HCl and HF from the hydrodehalogenation, and more preferably the entire effluent from the hydrodehalogenation of CF₃CCl₂CF₃ (including the CF₃CCl=CF₂, CF₃CH=CF₂, HCl and HF), with hydrogen over the hydrogenation catalyst. While the hydrogenation reaction proceeds even in the absence of HCl and HF, the HCl and HF present during the hydrogenation step moderates the hydrogenation reaction. In any case, in accordance with this invention, CF₃CH₂CHF₂ may be produced from CF₃CCl₂CF₃ without separation and removal of HCl and HF prior to CF₃CH₂CHF₂ production. In addition, passing the entire effluent from the hydrodehalogenation step on to the hydrogenation step avoids handling concerns associated with olefinic halogenated compounds as well as HCl and HF. The HCl and HF of the hydrogenation effluent is available for use along with other compounds thereof. For example, the HF is available for azeotropic combination with the fluorinated hydrocarbon compounds of the effluent from the hydrogenation reaction.

The contact of said hydrodehalogenation effluent with hydrogen in the presence of a hydrogenation catalyst and HCl and HF is suitably conducted at a temperature in the range of from 50°C to 300°C, and preferably from 50°C to 200°C. Contact time is typically from 5 to 100 seconds, preferably 10 to 30 seconds.

The molar ratio of hydrogen to CF₃CH=CF₂ in the hydrodehalogenation effluent typically is in the range from 1:1 to 50:1, and is preferably from 1.5:1 to 25:1, and more preferably from 2:1 to 10:1. Normally, at least 100 ppm each of HCl and HF is present; and typically for each mole of CF₃CH=CF₂, the hydrodehalogenation effluent also contains two moles of HCl and one mole of HF, especially when the entire effluent from the hydrodehalogenation step is passed to the hydrogenation step.

Hydrogen can be fed to the hydrodehalogenation and/or the hydrogenation steps either in the pure state or diluted with inert gas (e.g., nitrogen, helium or argon).

Alternatively, CF₃CH₂CHF₂ may be produced by reacting the CF₃CH=CF₂ and/or CF₃CCl=CF₂ hydrodehalogenation reaction product with hydrogen in an empty reaction vessel of nickel, iron or their alloys in accordance with the disclosure of U.S. Patent No. 5,364,992, which is incorporated herein in its entirety by reference.

The reaction products from the hydrogenation may be separated by conventional techniques, such as distillation. CF₃CH₂CHF₂ forms an azeotrope with HF (see PCT International Publication No. WO97/05089) and may also form an azeotrope with HCl; and conventional decantation/distillation may be employed if further purification of CF₃CH₂CHF₂ is desired. The azeotropic compositions of CF₃CH₂CHF₂ include a composition consisting essentially of from 84 to 44 mole percent HF and from 16 to 56 mole percent CF₃CH₂CHF₂ (which forms an azeotrope having a boiling point from - 50°C at 5.5 kPa to 130°C at 3853 kPa).

Pressure is not critical for the hydrofluorination, hydrogenation, and hydrodehalogenation processes described above. Atmospheric and superatmospheric pressures (e.g., pressure from 100 kPa to 7000 kPa) are the most convenient and are therefore preferred.

The hydrofluorination, hydrogenation and hydrodehalogenation reactions may be conducted in any suitable reactor. The reaction vessel should be constructed from materials which are resistant to the corrosive effects of hydrogen fluoride such as Inconel™ nickel alloy and Hastelloy™ nickel alloy.

The CF₃CCl₂CF₃ used as a reactant in this process may be produced by known art methods such as disclosed in U.S. Patent No. 5,057,634.

CF₃CH₂CHF₂ has numerous uses including applications in compositions used as refrigerants, blowing agents, propellants, cleaning agents, and heat transfer agents.

Without further elaboration, it is believed that one skilled in the art can, using the description herein, utilize the present invention to its fullest extent. The following specific embodiments are to be construed as illustrative, and not as constraining the remainder of the disclosure in any way whatsoever.

### EXAMPLES

### Hydrodehalogenation Catalyst Preparation for the Examples

Aqueous calcium nitrate (2.7 moles) is mixed with aqueous potassium fluoride (5.4 moles), heated and stirred briefly at 100°C to form a slurry of CaF₂. To this slurry is added copper nitrate (1 mole), nickel nitrate (1 mole) and chromium nitrate (1 mole) as solids. The slurry is stirred at 70 to 80°C until the salts, other than CaF₂, dissolve. This is followed by adding 0.1 mole of aqueous potassium hydroxide over 1 hour and boiling the mixture briefly. The slurry is cooled to 40 to 50°C and filtered. The solid is washed exhaustively to reduce the potassium content to an undetectable level. After drying, potassium hydroxide is added as a solution in quantities sufficient to provide a catalyst containing 9 weight % potassium. After drying again, the catalyst is calcined at 600°C for 8 to 16 hours, then granulated and screened to 1 to 2 mm particles. The catalyst is mixed with 1 to 5 wt% "Sterotex" powdered lubricant (registered trademark of Capital City Products Co., Columbus Ohio, division of Stokely-Van Camp, for its edible hydrogenated vegetable oil) to give 1/8" x 1/8" (3.2 mm x 3.2 mm) cylindrical pellets from a Stokes tablet machine.

### General Procedure for Product Analysis for the Examples

The products leaving the reactor were analyzed on line using a gas chromatograph. The column consisted of a 20' (6.1 m) x 1/8" (3.2 mm) stainless steel tube containing Krytox™ perfluorinated polyether on an inert support. Helium was used as the carrier gas. The product analyses are reported in mole%.

### Legend:

| | |
|---|---|
| 143a is CF₃CH₃ | 125 is CF₃CHF₂ |
| 216aa is CF₃CCl₂CF₃ | 226da is CF₃CHClCF₃ |
| 236fa is CF₃CH₂CF₃ | 235da is CF₃CHClCHF₂ |
| 225da is CF₃CHClCClF₂ | 245fa is CF₃CH₂CHF₂ |
| 254fb is CF₃CH₂CH₂F | 1225zc is CF₃CH=CF₂ |
| 1215xc is CF₃CCl=CF₂ | CT is contact time |
| HFP is CF₃CF=CF₂ | conv. is conversion |
| | sel. is selectivity |

### EXAMPLE 1

A 15" (381 mm) X 1/4" (6.4 mm) O.D. Inconel™ 600 nickel alloy U-tube reactor used for hydrodehalogenation (HDH) was charged with catalyst (21.7 g, 18 mL) pellets prepared substantially in accordance with the Catalyst Preparation described above. The HDH catalyst was in use for a variety of runs totaling 646 hours prior to its use in Example 1. Before starting the runs shown in Example 1, the HDH catalyst was regenerated with 50 sccm (8.3 x 10⁻⁷ m³/s) 50% air/50% N₂ at 350°C for 1 hour; 50 sccm (8.3 x 10⁻⁷ m³/s) air at 400°C for 2 hours; followed by purging with 200 sccm (3.3 x 10⁻⁶ m³/s) N₂ at 400°C for 40 minutes; and finally reduced with 50 sccm (8.3 x 10⁻⁷ m³/s) H₂ at 300°C for 75 minutes.

CF₃CCl₂CF₃ and H₂ were contacted with the catalyst at 350°C or 360°C and with a molar ratio of H₂:CF₃CCl₂CF₃ as shown in the table. Runs 1 to 3 were conducted at a pressure of 40 psig (380 kPa) and runs 4 to 9 were done at 100 psig (790 kPa). Results of the HDH reaction are shown in Table 1.

**TABLE 1**

| Run No. | HDH T(°C) | Molar Ratio H₂:216aa | CT Min. | % Conv. 216aa | %Sel. to | | | | |
|---|---|---|---|---|---|---|---|---|---|
| | | | | | 1225zc | 236fa | 245fa | 1215xc | 226da |
| 1 | 350 | 7 | 0.58 | 92.9 | 12.5 | 0.1 | 0.2 | 75.9 | 6.4 |
| 2 | 350 | 12 | 0.58 | 99.3 | 15.7 | 0.3 | 0.2 | 72.9 | 5.4 |
| 3 | 360 | 12 | 0.57 | 99.5 | 19.1 | 0.5 | 0.3 | 71.6 | 3.4 |
| 4 | 360 | 10 | 0.54 | 99.3 | 23.0 | 0.6 | 0.4 | 67.4 | 3.7 |
| 5 | 360 | 22 | 0.55 | 99.4 | 21.4 | 0.6 | 0.4 | 72.6 | 1.2 |
| 6 | 350 | 22 | 0.56 | 99.4 | 14.6 | 0.4 | 0.2 | 78.4 | 2.8 |
| 7 | 350 | 11 | 0.56 | 99.0 | 11.1 | 0.3 | 0.1 | 78.8 | 6.5 |
| 8 | 360 | 11 | 0.55 | 99.2 | 12.5 | 0.3 | 0.2 | 77.1 | 6.6 |
| 9 | 360 | 22 | 0.55 | 99.4 | 17.3 | 0.5 | 0.2 | 76.8 | 1.9 |
| ¹HDH is the hydrodehalogenation catalyst | | | | | | | | | |

### EXAMPLE 2

A second reactor for hydrogenation was added to the system on the exit side of the HDH reactor. This reactor consisted of an Inconel™ 600 tube 3/8" (9.5 mm) od. x 0.035" (0.9 mm) wall, 30" (762 mm) long packed with 21.8 grams (18.0 mL) of (0.5 weight% palladium on acid washed 12-30 mesh carbon (1.68-0.59 mm). The catalyst was treated with 50 sccm (8.3 x 10⁻⁷ m³/s) of hydrogen at 100°C for 15 hours prior to the run. The HDH reactor was the same as that used in Example 1. It was packed with 21.7 g of the HDH catalyst and treated with 50 sccm (8.3 x 10⁻⁷ m³/s) at 350°C for 15 hours prior to the run. The entire effluent of the HDH reactor (including HCl and HF) was passed into the second (hydrogenation) reactor. The contact time in the HDH reactor was 0.27 minutes except for runs 4 and 5 where it was 0.29 minutes. The temperature for the HDH reaction was 350°C for all the runs. The contact time (CT) shown in the table is for the Pd/C catalyst. The pressure of both reactors was 40 psig (377 kPa). Results of these reactions are shown in the Table 2.

**TABLE 2**

| Run No. | Pd/C T(°C) | Molar Ratio H₂:216aa | CT Min. | %Conv. 216aa | % Sel. to | | | | | |
|---|---|---|---|---|---|---|---|---|---|---|
| | | | | | 1225zc | 245fa | 254fb | 226da | 235da | 225da |
| 1 | 100 | 16 | 0.45 | 99.5 | 24.3 | 57.5 | <0.1 | 2.9 | 12.1 | 0.7 |
| 2 | 100 | 8 | 0.45 | 99.4 | 40.3 | 40.0 | 0.6 | 4.3 | 10.7 | 1.0 |
| 3 | 80 | 8 | 0.48 | 99.1 | 40.9 | 25.4 | 0.5 | 5.1 | 13.7 | 1.4 |
| 4 | 80 | 15 | 0.50 | 99.2 | 42.7 | 22.7 | 0.4 | 4.3 | 15.2 | 1.2 |
| 5 | 150 | 15 | 0.42 | 99.5 | 1.5 | 85.6 | 1.2 | 3.3 | 5.5 | 1.1 |
| 6 | 150 | 16 | 0.40 | 99.6 | 0.4 | 88.7 | 1.2 | 3.2 | 4.0 | 1.2 |
| 7 | 150 | 8 | 0.40 | 99.5 | 5.7 | 80.7 | 1.0 | 5.2 | 3.7 | 2.2 |
| 8 | 140 | 16 | 0.41 | 99.5 | 1.8 | 86.3 | 1.0 | 3.4 | 4.3 | 2.0 |
| 9 | 145 | 16 | 0.40 | 99.5 | 1.5 | 86.1 | 0.9 | 3.4 | 4.9 | 2.0 |
| 10 | 150 | 16 | 0.40 | 99.5 | 2.5 | 86.1 | 1.0 | 3.3 | 4.4 | 1.6 |
| 11 | 150 | 16 | 0.40 | 99.5 | 0.6 | 88.5 | 1.1 | 2.3 | 4.7 | 1.4 |

### EXAMPLE 3

A second reactor for hydrogenation was added to the system on the exit side of the HDH reactor. This reactor consisted of an Inconel™ 600 tube 3/8" (9.5 mm) od. x 0.035" (0.9 mm) wall, 30" (762 mm) long packed with 27.8 grams (35.9 mL) of (0.5 weight% palladium on acid washed 12-30 mesh carbon (1.68-0.59 mm). The catalyst was treated with 120 sccm (2.0 x 10⁻⁶ m³/s) of hydrogen at 150°C for one hour prior to the run. The HDH reactor was the same as that used in Example 1. It was packed with 21.7 g of the HDH catalyst and treated with 120 sccm (2.0 x 10⁻⁶ m³/s) of hydrogen at 350°C for one hour prior to the run. The entire effluent of the HDH reactor (including HCl and HF) was passed into the second (hydrogenation) reactor. The contact time in the HDH reactor was 0.14 minutes for run 1, 0.13 minutes for runs 2, 3 and 6, and 0.07 minutes for runs 4 and 5. The temperature for the HDH reaction was 350°C for run 1, 355°C for runs 2, 3, 4 and 6 and 360°C for run 5. The contact time (CT) shown in the table is for the Pd/C catalyst. The temperature of the hydrogenolysis reaction was 150°C for all runs. The pressure of both reactors was 40 psig (377 kPa). Results of these reactions are shown in the Table 3.

**TABLE 3**

| Run No. | Molar Ratio H₂:216aa | CT Min. | %Conv. 216aa | %Sel. to | | | | | | |
|---|---|---|---|---|---|---|---|---|---|---|
| | | | | 1225zc | 236fa | 245fa | 254fb | 226da | 235da | 225da |
| 1 | 16 | 0.25 | 100 | 0.6 | 6.1 | 87.9 | 1.6 | 1.3 | 0.8 | 0.2 |
| 2 | 16 | 0.25 | 100 | 0.5 | 5.9 | 88.1 | 1.6 | 1.5 | 1.0 | <0.1 |
| 3 | 16 | 0.25 | 100 | 0.4 | 6.5 | 86.0 | 1.7 | 2.1 | 1.8 | <0.1 |
| 4 | 16 | 0.13 | 100 | 0.5 | 19.7 | 68.9 | 1.4 | 6.4 | 1.6 | <0.1 |
| 5 | 16 | 0.13 | 100 | 0.4 | 18.0 | 69.1 | 1.4 | 7.5 | 2.0 | <0.1 |
| 11 | 16 | 0.25 | 100 | 0.3 | 10.2 | 77.7 | 1.7 | 5.4 | 3.2 | <0.1 |

### EXAMPLE 4

The reactor used for hydrodehalogenation (HDH) in Example 1 was charged with catalyst (24.1 g, 18.0 mL) 1/8" x 1/8" (3.2 mm x 3.2 mm) pellets prepared substantially in accordance with the Catalyst Preparation above. The HDH catalyst unlike that of Example 1 was not used previously, i.e., it was a fresh catalyst. Before starting the runs shown in Example 1, the HDH catalyst was treated with 110 sccm (1.8 x 10⁻⁶ m³/s) H₂ at 350°C and 40 psig (380 kPa) for one hour.

CF₃CCl₂CF₃ and H₂ were contacted with the catalyst at 350°C or 360°C and with a molar ratio of H₂:CF₃CCl₂CF₃ as shown in the table. All runs were conducted at a pressure of 40 psig (380 kPa). Results of the HDH reaction are shown in Table 4.

**TABLE 4**

| Run No. | HDH¹ T (°C) | Molar Ratio H₂:216aa | CT Min. | %Conv. 216aa | %Sel. to | | |
|---|---|---|---|---|---|---|---|
| | | | | | 1225zc | 1215xc | Other² |
| 1 | 350 | 16 | 0.27 | 100 | 9.1 | 87.4 | 3.6 |
| 2 | 350 | 16 | 0.27 | 100 | 10.2 | 86.2 | 3.7 |
| 3 | 350 | 8 | 0.27 | 100 | 12.0 | 84.0 | 4.0 |
| 4 | 350 | 16 | 0.27 | 100 | 10.9 | 84.8 | 4.2 |

| | | | | | | | |
|---|---|---|---|---|---|---|---|
| ¹HDH is the hydrodehalogenation catalyst | | | | | | | |
| ²Other includes 236fa, 245fa and 226da | | | | | | | |

### EXAMPLE 5

The reactor and catalyst used for hydrodehalogenation (HDH) in Example 3 was used. The HDH catalyst was treated prior to use with hydrogen at 300°C for one hour; 67% air/33% N₂ at 300°C for 1 hour; 50% air/50% N₂ at 400°C for 1 hour; air at 450°C for 2 hours; air at 500°C for 5 hours; air at 550°C for 5 hours; followed by purging with N₂ at 350°C for 3 hours; N₂/H₂ (1:1) at 350°C for one hour; and finally reduced with H₂ at 350°C for one hour.

CF₃CCl₂CF₃ and H₂ were contacted with the catalyst at 350°C and with a molar ratio of H₂:CF₃CCl₂CF₃ as shown in the Table 4. The HDH reaction was done at a pressure of 40 psig (380 kPa) and a contact time of 0.27 minutes. The entire effluent (including HCl and HF) was then passed into a second (hydrogenation) reactor which was the same as that used in Example 3 and contained the same catalyst. The hydrogenation catalyst was purged with hydrogen at 380 kPa and 150°C, the same temperature and pressure as the hydrogenation reaction, for 8 hours prior to use. The contact time of the hydrogenation reaction was 0.51 minutes. The results of the combined reactions are shown in Table 5.

**TABLE 5**

| Run No. | Molar Ratio H₂:216aa | %Conv. 216aa | %Sel. to | | | | | |
|---|---|---|---|---|---|---|---|---|
| | | | 1225zc | 245fa | 254fb | 226da | 235da | Other¹ |
| 1 | 16 | 100 | 0.0 | 89.0 | 2.7 | 0.1 | 4.6 | 3.6 |
| 2 | 8 | 100 | 3.8 | 80.6 | 2.1 | 1.0 | 9.2 | 3.3 |
| 3 | 8 | 100 | 4.8 | 81.3 | 2.0 | 1.2 | 7.6 | 2.9 |

| | | | | | | | | |
|---|---|---|---|---|---|---|---|---|
| ¹Other includes 236fa and 1225xc | | | | | | | | |

### EXAMPLE 6

The reactor and catalyst used for hydrodehalogenation (HDH) in Example 4 was used. The HDH catalyst was treated prior to use in the same way as the catalysts used in Example 4.

CF₃CCl₂CF₃ and H₂ were contacted with the catalyst at 350°C and with a molar ratio of H₂:CF₃CCl₂CF₃ as shown in the Table 4. The HDH reaction was done at a pressure of 40 psig (380 kPa) and a contact time of 0.27 minutes except for run 3 which was done at 0.13 minutes. The entire effluent (including HCl and HF) was then passed into a second (hydrogenation) reactor which was the same as that used in Example 3 and contained the same catalyst. The hydrogenation catalyst was purged with hydrogen at 380 kPa and 150°C, the same temperature and pressure as the hydrogenation reaction, for 8 hours prior to use. The contact time of the hydrogenation reaction was 0.51 minutes except for run 3 which was 0.25 minutes. The results of the combined reactions are shown in Table 6.

**TABLE 6**

| Run No. | Molar Ratio H₂:216aa | %Conv. 216aa | %Sel. to | | | | | |
|---|---|---|---|---|---|---|---|---|
| | | | 1225zc | 245fa | 254fb | 226da | 235da | Other¹ |
| 1 | 16 | 100 | 0.4 | 81.0 | 3.7 | 1.0 | 9.4 | 0.2 |
| 2 | 16 | 100 | 1.1 | 73.9 | 3.7 | 1.0 | 14.0 | 0.2 |
| 3 | 16 | 100 | 7.4 | 74.1 | 2.5 | 1.0 | 11.6 | 0.2 |
| 4 | 8 | 100 | 11.5 | 66.4 | 1.8 | 1.8 | 10.1 | 0.2 |

| | | | | | | | | |
|---|---|---|---|---|---|---|---|---|
| ¹Other includes 236fa and 1225xc | | | | | | | | |

### EXAMPLE 7

### CF₃CH=CF₂ + HF → CF₃CH₂CF₃ (Liquid Phase)

A Hastelloy™ C nickel alloy (160 mL) autoclave equipped with a magnetically driven stirrer, pressure transducer, thermocouple well, and vapor inlet valve was evacuated, cooled in liquid nitrogen, and charged with anhydrous HF (50 g, 2.5 moles). 1,1,3,3,3-Pentafluoro-1-propene (20.2 g, 0.15 mole) was added to the autoclave at a temperature of about -31°C from a small cylinder. The autoclave was then warmed to ambient temperature. The contents of the autoclave were stirred at about 500 rpm and the autoclave was heated to 70°C over the course of about 8 minutes. The pressure rose to 106 psig (832 kPa) and then leveled out at 99 psig (784 kPa). After 2 hours at 71 °C and 99 psig (784 kPa), a sample of the vapor phase of the autoclave was analyzed by GC-MS (see Table 7).

**TABLE 7**

| Component | GC Area % |
|---|---|
| C₃F₆ | 0.3 |
| 1225zc | 84.9 |
| 236fa | 14.3 |

### EXAMPLE 8

### CF₃CH=CF₂ + HF → CF₃CH₂CF₃ (Vapor Phase)

A 15 mL tubular reactor was packed with 13.7 g of a catalyst comprising a 0.02:0.98:1.0 ratio of CoF₂, ZnF₂, and AlF₃, respectively. The catalyst was activated by heating to 250°C over the course of 1 hour while passing 50 sccm (8.3 x 10⁻⁷ m³/s) nitrogen through the reactor. The nitrogen flow was then reduced to 20 sccm (3.3 x 10⁻⁷ m³/s) and HF was admitted at 50 sccm (8.3 x 10⁻⁷ m³/s) at 250°C for 1 hour.

A 4:1 molar mixture of HF and 1,1,3,3,3-pentafluoro-1-propene was fed to the reactor with a catalyst contact time of 15 seconds at 300°C. A comparison of the reactor feed gas and effluent under these conditions is given in Table 8.

**TABLE 8**

| Temp. °C | 143a | HFP | 1225zc | 236fa | 125 |
|---|---|---|---|---|---|
| Feed | 0.0 | 0.2 | 98.4 | 0.6 | 0.0 |
| 350 | 0.2 | 0.1 | 1.4 | 97.8 | 0.1 |
| 300 | 0.1 | 0.1 | 0.4 | 98.9 | 0.0 |

Other products observed by GC include CH₃Cl, CH₃F, CHF₂CClF₂, and CHCIFCF₃ which appear to be contaminants in the 1225zc.

## Claims

1. A process for producing the pentafluoropropenes of the formula CF₃CX=CF₂, where X is H or Cl, comprising:
hydrodehalogenating CF₃CCl₂CF₃ with hydrogen at an elevated temperature in the vapor phase over a catalyst comprising at least one component selected from the group consisting of elemental metals, metal oxides, metal halides and metal oxyhalides; wherein the metal of said hydrodehalogenation catalyst component is selected from copper, nickel, chromium and mixtures thereof and the halogen of said halides and said oxyhalides is selected from fluorine, chlorine and mixtures thereof.

2. The process of Claim 1 wherein the catalyst contains proportionally 1.0 mole CuO, 0.2 to 1.0 mole NiO, 1 to 1.2 moles Cr₂O₃ on 1.3 to 2.7 moles CaF₂, and is promoted with 1 to 20 weight %, based on the total catalyst weight, of an alkali metal selected from K, Cs, and Rb.

3. The process of Claim 2 wherein the catalyst is promoted with from 2 to 15 weight % K, based on the total catalyst weight.

4. The process of Claim 1 wherein the catalyst contains proportionally 1.0 mole CuO, 0.2 to 1.0 mole NiO, 1 to 1.2 moles Cr₂O₃, 0.4 to 1.0 mole MoO₃, and 0.8 to 4.0 mole CaF₂.

5. The process of Claim 4 wherein the catalyst is promoted with at least one compound selected from the group consisting of MgF₂, MnF₂, and BaF₂.

6. The process of Claim 1 wherein the catalyst consisting essentially of copper, nickel and chromium oxides, promoted with potassium salt, on calcium fluoride.

7. A process for producing CF₃CH₂CHF₂, comprising:
(a) hydrodehalogenating CF₃CCl₂CF₃ with hydrogen in accordance with the process of Claim 1 to produce a product comprising CF₃CCl=CF₂, CF₃CH=CF₂, HCl and HF; and
(b) reacting at least one of said CF₃CCl=CF₂ and CF₃CH=CF₂ produced in (a) in the vapor phase with hydrogen to produce CF₃CH₂CHF₂.

8. The process of Claim 7 wherein in (b) at least one of said CF₃CCl=CF₂ and CF₃CH=CF₂ is contacted with hydrogen in the presence of a hydrogenation catalyst and in the presence of HCl and HF.

9. The process of Claim 8 wherein the entire effluent from (a) is passed on to (b).

10. The process of Claim 8 wherein CF₃CH₂CHF₂ is produced from CF₃CCl₂CF₃ without separation and removal of HCl and HF prior to CF₃CH₂CHF₂ production.

11. A process for producing CF₃CHFCF₃, comprising:
(a) hydrodehalogenating CF₃CCl₂CF₃ with hydrogen in accordance with the process of Claim 1 to produce a product comprising CF₃CCl=CF₂, CF₃CH=CF₂, HCl and HF; and
(b) reacting the CF₃CCl=CF₂ produced in (a) with HF to produce CF₃CHFCF₃.

12. A process for producing CF₃CH₂CF₃, comprising:
(a) hydrodehalogenating CF₃CCl₂CF₃ with hydrogen in accordance with the process of Claim 1 to produce a product comprising CF₃CCl=CF₂, CF₃CH=CF₂, HCl and HF; and
(b) reacting the CF₃CH=CF₂ produced in (a) with HF to produce CF₃CH₂CF₃.

## Patentansprüche

1. Verfahren zum Herstellen der Pentafluorpropene der Formel CF₃CX=CF₂, wobei X H oder Cl ist, umfassend:
Hydrohalogenieren von CF₃CCl₂CF₃ mit Wasserstoff bei einer erhöhten Temperatur in der Dampfphase über einem Katalysator, umfassend mindestens eine Komponente, ausgewählt aus der Gruppe, bestehend aus elementaren Metallen, Metalloxiden, Metallhalogeniden und Metalloxyhalogeniden, wobei das Metall der Hydrohalogenierungskatalysatorkomponente aus Kupfer, Nickel, Chrom und Gemischen davon ausgewählt ist und das Halogen der Halogenide und der Oxyhalogenide aus Fluor, Chlor und Gemischen davon ausgewählt ist.

2. Verfahren nach Anspruch 1, wobei der Katalysator proportional 1,0 mol CuO, 0,2 bis 1,0 mol NiO, 1 bis 1,2 mol Cr₂O₃ auf 1,3 bis 2,7 mol CaF₂ enthält und mit 1 bis 20 Gew.-%, bezogen auf das gesamte Katalysatorgewicht, eines Alkalimetalls, ausgewählt aus K, Cs und Rb, promotiert ist.

3. Verfahren nach Anspruch 2, wobei der Katalysator mit von 2 bis 15 Gew.-% K, bezogen auf das gesamte Katalysatorgewicht, promotiert ist.

4. Verfahren nach Anspruch 1, wobei der Katalysator proportional 1,0 mol CuO, 0,2 bis 1,0 mol NiO, 1 bis 1,2 mol Cr₂O₃, 0,4 bis 1,0 mol MoO₃ und 0,8 bis 4,0 mol CaF₂ enthält.

5. Verfahren nach Anspruch 4, wobei der Katalysator mit mindestens einer Verbindung, ausgewählt aus der Gruppe bestehend aus MgF₂, MnF₂ und BaF₂, promotiert ist.

6. Verfahren nach Anspruch 1, wobei der Katalysator im wesentlichen aus Kupfer-, Nickel- und Chromoxiden, promotiert mit Kaliumsalz, auf Calciumfluorid besteht.

7. Verfahren zum Herstellen von CF₃CH₂CHF₂, umfassend:
(a) Hydrohalogenieren von CF₃CCl₂CF₃ mit Wasserstoff nach dem Verfahren von Anspruch 1, um ein Produkt, umfassend CF₃CCl=CF₂, CF₃CH=CF₂, HCl und HF, herzustellen; und
(b) Umsetzen von mindestens einem von den CF₃CCl=CF₂ und CF₃CH=CF₂, hergestellt in (a), in der Dampfphase mit Wasserstoff, um CF₃CH₂CHF₂ herzustellen.

8. Verfahren nach Anspruch 7, wobei in (b) mindestens eines von den CF₃CCl=CF₂ und CF₃CH=CF₂ in Anwesenheit eines Hydrierungskatalysators und in Anwesenheit von HCl und HF in Kontakt mit Wasserstoff gebracht wird.

9. Verfahren nach Anspruch 8, wobei der gesamte Abstrom von (a) zu (b) weitergeleitet wird.

10. Verfahren nach Anspruch 8, wobei CF₃CH₂CHF₂ aus CF₃CCl₂CF₃ ohne Abtrennung und Entfernung von HCl und HF vor der CF₃CH₂CHF₂-Herstellung hergestellt wird.

11. Verfahren zum Herstellen von CF₃CHFCF₃, umfassend:
(a) Hydrohalogenieren von CF₃CCl₂CF₃ mit Wasserstoff nach dem Verfahren von Anspruch 1, um ein Produkt, umfassend CF₃CCl=CF₂, CF₃CH=CF₂, HCl und HF, herzustellen; und
(b) Umsetzen des CF₃CCl=CF₂, hergestellt in (a), mit HF, um CF₃CHFCF₃ herzustellen.

12. Verfahren zum Herstellen von CF₃CH₂CF₃, umfassend:
(a) Hydrohalogenieren von CF₃CCl₂CF₃ mit Wasserstoff nach dem Verfahren von Anspruch 1, um ein Produkt, umfassend CF₃CCl=CF₂, CF₃CH=CF₂, HCl und HF, herzustellen; und
(b) Umsetzen des CF₃CH=CF₂, hergestellt in (a), mit HF, um CF₃CH₂CF₃ herzustellen.

## Revendications

1. Procédé de production de pentafluoropropènes de formule CF₃CX=CF₂, où X est H ou Cl, comprenant :
l'hydrodéshalogénation de CF₃CCl₂CF₃ avec de l'hydrogène à une température élevée dans la phase vapeur au-dessus d'un catalyseur comprenant au moins un composant choisi parmi le groupe constitué des métaux élémentaires, des oxydes métalliques, des halogénures métalliques et des oxyhalogénures métalliques ; dans lequel le métal dudit composant de catalyseur d'hydrodéshalogénation est choisi parmi le cuivre, le nickel, le chrome et les mélanges de ceux-ci et l'halogène desdits halogénures et desdits oxyhalogénures est choisi parmi le fluor, le chlore et les mélanges de ceux-ci.

2. Procédé selon la revendication 1 dans lequel le catalyseur contient proportionnellement 1,0 mole de CuO, 0,2 à 1,0 mole de NiO, 1 à 1,2 mole de Cr₂O₃ sur 1,3 à 2, 7 moles de CaF₂ et est promu avec 1 à 20% en poids, par rapport au poids total du catalyseur, d'un métal alcalin choisi parmi K, Cs et Rb.

3. Procédé selon la revendication 2 dans lequel le catalyseur est promu avec 2 à 15% en poids de K par rapport au poids total du catalyseur.

4. Procédé selon la revendication 1 dans lequel le catalyseur contient proportionnellement 1,0 mole de CuO, 0,2 à 1,0 mole de NiO, 1 à 1,2 mole de Cr₂O₃, 0,4 à 1,0 mole de MoO₃ et 0,8 à 4,0 moles de CaF₂.

5. Procédé selon la revendication 4 dans lequel le catalyseur est promu avec au moins un composé choisi parmi le groupe constitué de MgF₂, MnF₂ et BaF₂.

6. Procédé selon la revendication 1 dans lequel le catalyseur constitué essentiellement d'oxydes de cuivre, de nickel et de chrome, est promu avec un sel de potassium, sur du fluorure de calcium.

7. Procédé de production de CF₃CH₂CHF₂ comprenant :
(a) l'hydrodéshalogénation de CF₃CCl₂CF₃ avec de l'hydrogène selon le procédé de la revendication 1 pour produire un produit comprenant CF₃CCl=CF₂, CF₃CH=CF₂, HCl et HF ; et
(b) la réaction d'au moins un desdits CF₃CCl=CF₂ et CF₃CH=CF₂ produits en (a) dans la phase vapeur avec de l'hydrogène pour produire CF₃CH₂CHF₂.

8. Procédé selon la revendication 7 dans lequel dans (b) au moins un desdits CF₃CCl=CF₂ et CF₃CH=CF₂ est mis en contact avec de l'hydrogène en présence d'un catalyseur d'hydrogénation et en présence de HCl et HF.

9. Procédé selon la revendication 8 dans lequel l'effluent entier de (a) est passé sur (b).

10. Procédé selon la revendication 8 dans lequel CF₃CH₂CHF₂ est produit à partir de CF₃CCl₂CF₃ sans séparation et élimination de HCl et HF avant la production de CF₃CH₂CHF₂.

11. Procédé de production de CF₃CHFCF₃ comprenant :
(a) l'hydrodéshalogénation de CF₃CCl₂CF₃ avec de l'hydrogène selon le procédé de la revendication 1 pour produire un produit comprenant CF₃CCl=CF₂, CF₃CH=CF₂, HCl et HF ; et
(b) la réaction de CF₃CCl=CF₂ produit en (a) avec HF pour produire CF₃CHFCF₃.

12. Procédé de production de CF₃CH₂CF₃ comprenant :
(a) l'hydrodéshalogénation de CF₃CCl₂CF₃ avec de l'hydrogène selon le procédé de la revendication 1 pour produire un produit comprenant CF₃CCl=CF₂, CF₃CH=CF₂, HCl et HF ; et
(b) la réaction du CF₃CH=CF₂ produit en (a) avec HF pour produire CF₃CH₂CF₃.
